# FASCICULE DE BREVET EUROPEEN

(11) **EP 1 331 021 B1**
(45) Date de publication et mention de la délivrance du brevet: **26.10.2005**
(21) Numéro de dépôt: 03290189.4
(22) Date de dépôt: 24.01.2003
(51) Int. Cl.: A61N 1/05

(54) **Nécessaire de mise en place d'une sonde intracardiaque de stimulation ou de défibrillation du type à vis rétractable**
Einführungsvorrichtung für eine Stimulations- oder Defibrillationsleitung mit einer einschraubbaren Schraubwendel
Introduction tool for an endocardial stimulation or defibrillation lead having a retractable fixation helix

(30) Priorité: 25.01.2002 FR 0200946
(43) Date de publication de la demande: 30.07.2003
(73) Titulaire: ELA MEDICAL, 92541 Montrouge (FR)
(72) Inventeur: Ollivier, Jean-François, 91190 Villiers le Bacle (FR); Bessoule, Frédéric, 91600 Savigny sur Orge (FR)
(74) Mandataire: Dupuis-Latour, Dominique

(56) Documents cités:
- FR-A- 2 724 566
- US-A- 4 646 755
- US-A- 5 129 404
- US-A- 5 522 875

## Description

L'invention concerne la mise en place des sondes intracardiaques de stimulation ou de défibrillation.

Ces sondes, implantées dans une cavité du myocarde, permettent le recueil de signaux de dépolarisation, l'application d'impulsions de stimulation et/ou d'un choc de défibrillation ou de cardioversion produits par un stimulateur cardiaque, défibrillateur, cardioverteur et/ou dispositif de type "multisite" ou, plus généralement, par un "dispositif médical implantable actif" tel que défini par la Directive 90/385/CEE du 20 juin 1990 du Conseil des communautés européennes.

L'invention concerne plus précisément la mise en place des sondes dites "à vis" (vis rétractable ou non), qui sont pourvues à leur extrémité distale d'une vis permettant l'ancrage dans le tissu de l'endocarde au point de contact avec ce dernier.

Le EP-A-0 591 053 décrit une telle sonde, actuellement commercialisée par ELA Médical sous la dénomination *Stelix* (marque déposée).

De façon générale, une sonde de stimulation ou de défibrillation est constituée d'un "corps de sonde" formé d'une gaine (tube creux flexible) terminée à son extrémité distale par une tête de sonde portant l'électrode ou les électrodes destinées à venir en contact avec le myocarde. Le corps de sonde est relié à son extrémité proximale à une fiche de connexion électrique raccordable à une tête de connecteur d'un stimulateur ou d'un défibrillateur, le tube du corps de sonde comportant un ou plusieurs conducteurs reliant électriquement les bornes de la fiche de connexion à l'électrode ou aux électrodes de la tête.

Dans le cas d'une sonde à vis rétractable, la tête de sonde est en outre pourvue d'un mécanisme permettant d'escamoter la vis de manière à protéger les parois de la veine pendant l'introduction de la sonde jusqu'à ce que l'extrémité de la tête de sonde vienne en butée contre la paroi de l'endocarde. Une fois cette position atteinte, le chirurgien manipule alors la sonde de manière à déplacer la vis suivant un double mouvement, d'abord de translation axiale pour déployer la vis hors du logement de la tête de sonde où elle se trouvait, puis de rotation de cette vis pour réaliser son ancrage dans la paroi de l'endocarde.

Dans le cas de la sonde décrite par le EP-A-0 591 053 précité, ce double mouvement de déploiement et de rotation est réalisé de la manière suivante.

Dans un premier temps, le chirurgien introduit dans le tube creux de la sonde un premier mandrin ou stylet de type "mandrin-tournevis", c'est-à-dire dont l'extrémité distale présente une forme aplatie. Ce méplat vient coopérer avec un organe homologue du mécanisme de déploiement de la vis à l'intérieur du corps de sonde, et ce déploiement est obtenu en maintenant fixe, d'une main, le mandrin-tournevis, et en faisant tourner, de l'autre main, dans le sens des aiguilles d'une montre le corps de sonde (par la partie proximale de la gaine), sur cinq à six tours complets.

Une fois la vis déployée, le praticien retire le mandrin-tournevis et insère dans le tube un autre mandrin, dont l'extrémité est pourvue d'un renflement qui a pour fonction, à l'intérieur de la tête de sonde, de rendre la vis solidaire du corps de sonde (alors que, dans l'étape précédente de déploiement, ces deux éléments étaient désolidarisés pour permettre le déploiement de la vis). Le praticien procède alors à l'ancrage de la vis en faisant, à nouveau, tourner le corps de sonde dans le sens des aiguilles d'une montre sur cinq à six tours, l'extrémité proximale du mandrin étant maintenue fixe. La tête de sonde est munie d'un système de limitation de couple qui minimise la contrainte de torsion lors du vissage, de manière à ne pas endommager les tissus et éviter toute déformation ou rupture de la vis. En d'autres termes, si le couple résistant lors du vissage dans l'endocarde dépasse un seuil donné, la vis n'est plus entraînée par la rotation du corps de sonde.

Ce système est efficace et sûr. Il est en outre réversible, le chirurgien pouvant dévisser la vis et la rétracter par une manoeuvre inverse de celle décrite.

Il présente toutefois plusieurs inconvénients :
- tout d'abord, il nécessite l'usage de deux mandrins différents, ce qui complique le mode opératoire car il est nécessaire de retirer le mandrin-tournevis pour pouvoir insérer le mandrin renflé.
- d'autre part, comme on l'a indiqué plus haut, la manoeuvre tant de déploiement que d'ancrage est réalisée par la rotation du corps de sonde (par la partie proximale de la gaine) et non du mandrin. Ce dernier doit rester fixe, sa rotation étant empêchée par une poignée de maintien tenue par le chirurgien. Mais si, au lieu de tourner le corps de sonde, le chirurgien tourne le mandrin (comme cela est très souvent le cas avec d'autres types de sonde), la gaine va former des boucles à l'intérieur de la cavité cardiaque, risquant ainsi de déplacer la tête de sonde du fait de l'élasticité de cette gaine, avant ou pendant l'ancrage de la tête. Cet inconvénient est encore accentué lorsque, pour un meilleur placement de la tête de sonde, le chirurgien a préalablement déformé le mandrin en le coudant à quelques centimètres de la tête de sonde, afin de mieux se conformer à la configuration de la cavité cardiaque : dans ce cas, toute rotation, même faible, du mandrin aura tendance à provoquer une rotation du corps de sonde autour du coude, au lieu de transmettre la rotation axialement à la tête de sonde.
- également, le frottement de la gaine sur la paroi de la veine produit pendant le mouvement de rotation une accumulation de contraintes qui se libèrent soudainement en provoquant des à-coups dans le mouvement, très gênants pour le chirurgien.

De nombreux autres types de sondes à vis rétractable ont été proposés, par exemple par le US-A-4 106 512, ou encore le US-A-5 129 404 qui décrit en outre un dispositif d'implantation adapté. Mais ces dispositifs soit utilisent un outil spécial relativement complexe pour réaliser le mouvement combiné de déploiement et de vissage, soit imposent d'imprimer un mouvement de rotation à la gaine du corps de sonde, avec toujours le risque de former des boucles et de déplacer le point d'implantation avant que la tête de sonde n'ait pu être ancrée dans l'endocarde.

Le but de l'invention est de remédier aux inconvénients précités, en proposant un nécessaire de mise en place d'une sonde à vis qui permette, en un seul mouvement et avec un seul et même outil, de réaliser le déploiement de la vis (dans le cas d'une vis rétractable) et l'ancrage de celle-ci dans le myocarde, et ceci par rotation d'une poignée de manoeuvre d'un mandrin, donc sans rotation de la gaine du corps de sonde.

Un autre but de l'invention est de proposer un tel nécessaire qui procure, par lui-même, une limitation du couple d'entraînement de la vis, permettant d'éviter tout endommagement des tissus et/ou de la vis, et ceci sans qu'il soit nécessaire de recourir à un mécanisme particulier de limitation de couple incorporé à la tête de sonde.

Cette dernière propriété permet en particulier au nécessaire de l'invention d'être utilisable avec une très grande variété de sondes à vis, que la vis soit rétractable ou non.

De plus, comme on le verra, le mécanisme de l'invention reste totalement réversible, permettant donc, si nécessaire, une modification du point d'implantation et un retrait de la sonde sans aucun risque pour le patient.

À cet effet, l'invention propose un nécessaire de mise en place d'une sonde intracardiaque de stimulation ou de défibrillation du type sonde à vis. Cette sonde est d'un type comparable à celle décrite dans le EP-A-0 591 053 précité, comportant un corps de sonde avec une gaine creuse souple pourvue à son extrémité distale d'une tête de sonde comportant au moins une électrode de stimulation et une vis d'ancrage, la tête de sonde étant apte à être entraînée en rotation pour permettre l'ancrage de la vis par vissage dans la paroi de l'endocarde.

Ce nécessaire comporte, de manière en elle-même connue, un mandrin amovible, apte à être introduit à l'intérieur de la gaine du corps de sonde et mobile en translation et en rotation à l'intérieur de celle-ci, ce mandrin comportant à son extrémité distale un moyen de couplage en rotation pour permettre ledit entraînement en rotation de la tête de sonde.

Selon l'invention, le mandrin comprend : une âme centrale portant à son extrémité distale ledit moyen de couplage en rotation, et à son extrémité proximale un organe de manipulation ; et un tube creux logeant axialement l'âme, la longueur de ce tube creux étant inférieure à celle de l'âme, de sorte que cette dernière émerge du tube creux aux deux extrémités, proximale et distale, du tube, de manière à permettre l'entraînement en rotation de l'âme et corrélativement du moyen de couplage en rotation, par action sur l'organe de manipulation, essentiellement sans mise en rotation du tube creux.

Le moyen de couplage en rotation peut notamment être un méplat formé à l'extrémité de l'âme.

Très avantageusement, le matériau de l'âme est choisi de manière que le moment de torsion susceptible d'être transmis par l'âme soit supérieur au couple minimal d'entraînement du mécanisme de manoeuvre de la tête sur la totalité de la course de ce mécanisme, et inférieur au couple résistant opposé par la tête de sonde après ancrage de la vis dans la paroi de l'endocarde. De cette manière, en fin de course, après un nombre prédéterminé de tours correspondant à la manoeuvre complète de déploiement et de vissage de la vis d'ancrage, toute rotation supplémentaire de l'âme se traduit par un vrillage de celle-ci à l'intérieur du tube, essentiellement sans augmentation du couple transmis au moyen de couplage en rotation.

De préférence, l'âme centrale et le tube creux sont relativement rigides par rapport à la gaine du corps de sonde, et plastiquement déformable localement.

L'organe de manipulation peut être une poignée de manipulation solidaire de l'âme à son extrémité proximale. En variante, il peut être prévu un outil de manipulation comportant d'une part une poignée, formant ledit organe de manipulation, pourvue de moyens propres à pincer localement l'âme de manière à solidariser cette dernière à la poignée, et d'autre part un manche apte à être solidarisé à la gaine du corps de sonde et au tube creux .

Dans une variante avantageuse, la poignée inclut des moyens moteurs aptes à permettre l'entraînement en rotation, par rapport au manche, desdits moyens propres à pincer localement l'âme ; ces moyens moteurs peuvent notamment être des moyens à ressort élastique, la poignée incluant un remontoir apte à mettre en tension ces moyens à ressort, ainsi que des moyens de limitation du couple applicable à l'âme lors de la manoeuvre du remontoir.

Document FR2724566 décrit un dispositif de manoeuvre motorisée d'une sonde cardiaque à électrode comme décrit dans le préambule de la revendication 1.

On va maintenant décrire un exemple de mise en oeuvre de l'invention, en référence aux dessins annexés.
La figure 1 montre, en position rétractée et en position déployée, une tête de sonde du type à vis rétractable.
La figure 2 illustre le corps de sonde en configuration d'implantation.
La figure 3 illustre le mandrin spécifique proposé par l'invention.
La figure 4 représente la caractéristique du couple d'entraînement en fonction du nombre de tours lors de la manoeuvre du mandrin selon l'invention.
Les figures 5 et 6 montrent, respectivement en perspective et en coupe longitudinale, un outil pouvant faire partie du nécessaire de l'invention, pour la manoeuvre du mandrin de la figure 3.
La figure 7 montre, en coupe longitudinale, une variante avantageuse de la poignée de l'outil de la figure 6.

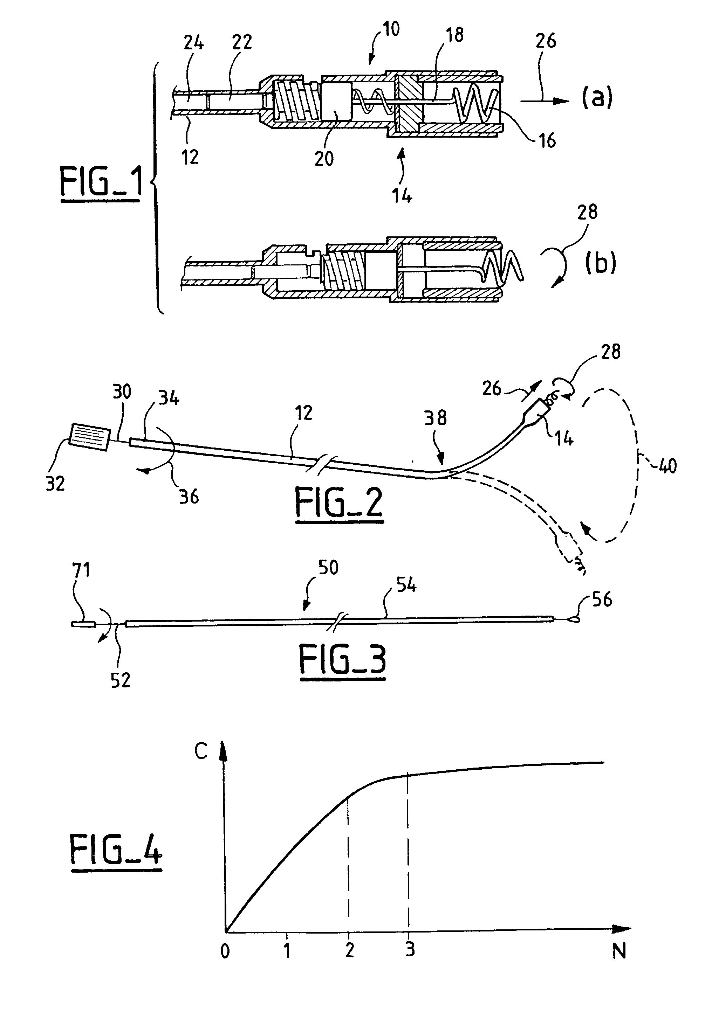

Sur la figure 1, on a représenté une tête de sonde du type à vis rétractable, avec la vis en position rétractée (figure 1a) et en position déployée (figure 1b).

Cette tête de sonde 10 est montée à l'extrémité d'une gaine 12 avec laquelle elle constitue le corps de sonde. La gaine 12 a la forme d'un tube creux flexible incorporant un ou plusieurs conducteur(s) électrique(s), non représentés.

La tête de sonde comporte un mécanisme 14 permettant de déployer une vis 16 destinée à venir s'ancrer dans la paroi de l'endocarde, pour assurer une liaison mécanique, et éventuellement électrique (suivant le type de vis, isolée ou non), avec le tissu du myocarde et empêcher tout déplacement ou délogement de la tête de sonde. Cette vis est placée à l'extrémité d'une tige 18 solidaire d'un piston 20 mobile à l'intérieur de la tête de sonde. Le piston 20 est prolongé en direction proximale par une tige 22, qui peut être entraînée en rotation par un mandrin-tournevis introduit dans la lumière 24 de la gaine 12.

La manoeuvre consiste, dans un premier temps, à déployer la vis hors du logement de la tête de sonde où elle se trouve initialement (position de la figure 1a), par un mouvement axial, dans le sens distal, par rapport à la tête de sonde (flèche 26). Dans un second temps (figure 1b), l'ensemble du corps de sonde, incluant donc la vis, est entraîné en rotation (flèche 28 de la figure 1b), pour réaliser le vissage de la vis déployée, de manière à permettre l'ancrage de cette dernière dans la paroi de l'endocarde. Dans le cas d'une vis à sonde non rétractable, seule l'étape correspondant à la figure 1b est réalisée.

Comme cela a été décrit plus haut, dans la technique utilisée jusqu'à présent, la première étape est réalisée au moyen d'un mandrin-tournevis introduit dans la lumière 24 permettant de faire tourner le piston 20 par rapport à l'enveloppe de la tête de sonde 10, tandis que la seconde étape est réalisée avec un mandrin renflé permettant de faire tourner l'ensemble du corps de sonde.

On a illustré figure 2 le corps de sonde 12 dans lequel a été introduit l'un de ces mandrins, référencé 30, terminé à son extrémité proximale par une poignée de maintien 32.

Comme on l'a indiqué plus haut, aussi bien le déploiement de la vis que son vissage dans la paroi de l'endocarde (mouvements 26 et 28, respectivement) sont obtenus en faisant tourner l'extrémité proximale 34 du corps de sonde 12 (flèche 36) en maintenant fixe le mandrin 30. En d'autres termes, le chirurgien tient d'une main la poignée 32 en l'immobilisant et, de l'autre main, fait tourner l'extrémité proximale 34 du corps de sonde.

Ce mouvement est moins naturel que celui consistant à immobiliser l'extrémité proximale 34 du corps de sonde en faisant tourner la poignée du mandrin 32. Mais surtout une rotation, même faible, du mandrin peut avoir pour effet un déplacement important de la tête de sonde lorsque le mandrin est un mandrin déformable, en matériau relativement rigide, qui a été volontairement coudé (en 38 sur la figure 2) par le chirurgien, de façon à permettre une meilleure orientation générale et faciliter son positionnement à l'endroit voulu de la cavité cardiaque. Toute rotation, même faible du mandrin 30 aura alors pour effet d'entraîner en rotation (flèche 40) la partie de la sonde située distalement par rapport au coude 38, risquant ainsi un délogement de la sonde pendant sa mise en place.

L'invention propose une autre technique de mise en place, utilisant un seul et unique mandrin de type "mandrin-tournevis", mais ne présentant pas les inconvénients qui ont été exposés ci-dessus.

Le mandrin de l'invention est utilisable avec tout type de sonde dont le déploiement de la vis et le vissage peuvent être commandés par un seul et même mouvement de rotation, obtenu au moyen d'un mandrin-tournevis, c'est-à-dire pourvu d'un embout en forme de méplat.

Le mandrin selon l'invention est illustré figure 3.

Ce mandrin 50 est en fait constitué de deux éléments coaxiaux, avec une âme centrale 52 logée dans un tube creux 54 dont le diamètre externe est comparable à celui d'un mandrin traditionnel formé d'un simple fil massif, par exemple d'un diamètre de 1 French (0,33 mm), donc un diamètre compatible avec le diamètre intérieur de la lumière de la gaine, sans modification.

L'âme centrale 52 présente une longueur supérieure à celle du tube creux 50, de manière à émerger aux deux extrémités de celui-ci. Côté proximal, l'âme 52 est terminée par un tube soudé 71 de plus fort diamètre, pour permettre une manipulation plus aisée. L'extrémité distale de l'âme 52 porte un méplat 56 qui permettra un couplage à un organe de manoeuvre homologue (fente ou analogue) de la tête de sonde pour permettre la manoeuvre de la vis d'ancrage.

Pour réaliser cette opération, le chirurgien introduit le mandrin 50 dans la lumière 24 de la gaine 12 du corps de sonde et manoeuvre la vis par rotation de l'âme centrale 52 - et de cette âme seulement, sans rotation du tube externe 54.

L'âme 52 et le tube creux 54 peuvent être réalisés dans un matériau plastiquement déformable, de manière que le chirurgien puisse donner une forme coudée à l'ensemble gaine-mandrin préalablement à l'introduction dans le réseau veineux. Pendant son cheminement dans ce dernier, l'ensemble se redresse du fait de l'élasticité des matériaux mais, une fois atteinte la cavité cardiaque, le coude se reforme, permettant ainsi au chirurgien de mieux positionner l'électrode à l'endroit voulu de la cavité. Dans la mesure où la manoeuvre de la vis est obtenue par la rotation de l'âme centrale 52 sans rotation du tube creux 54, la présence d'un coude n'est en aucune façon gênante, puisque le corps de sonde ne peut en aucune façon être entraîné par l'âme centrale, du fait de l'interposition du tube creux 54.

La rotation libre de l'âme 52 dans le tube creux 54 est assurée par un choix des dimensions des diamètres intérieurs et extérieurs ainsi que par l'état d'écrouissage du matériau. On évite de cette façon l'effet de ressaut du mandrin dans le tube, qui évite les à-coups de rotation.

Un autre avantage de l'invention réside dans la possibilité de disposer d'une fonction de limitation du couple de rotation, sans qu'il soit besoin de prévoir de mécanisme spécifique dans la tête de sonde.

En effet, dans la mesure où le couple de rotation est transmis par l'âme centrale 52 dont le diamètre est très faible, dès que le couple résistant sur la vis dépasse le moment de torsion maximal de l'âme centrale 52, aucun couple supplémentaire ne peut être transmis ; il sera absorbé par l'âme 52 qui se vrillera à l'intérieur du tube creux 54.

La figure 4 illustre la caractéristique du couple C en fonction du nombre de tours N imprimés à l'âme centrale 52 : jusqu'à 2 ou 3 tours, correspondant à un mouvement complet de déploiement et de vissage de la vis, le couple augmente progressivement. Lorsque la vis est ancrée à fond dans le myocarde, même si le chirurgien continue son mouvement de rotation, l'âme centrale se vrille à l'intérieur du tube creux, limitant ainsi le couple appliqué à la vis, en évitant toute rupture ou torsion de cette dernière et tout endommagement des tissus du myocarde.

Par ailleurs, la limite de déformation élastique du matériau constituant l'âme centrale est choisie pour que, si la rotation est poursuivie, l'âme se vrille dans un mouvement de déformation, typiquement sur plusieurs dizaines de tours, sans aller jusqu'à la rupture. Ceci permet de conserver une réversibilité complète, une rotation en sens contraire de l'âme centrale autorisant le dévissage et la rétractation de la vis à l'intérieur de la tête de sonde.

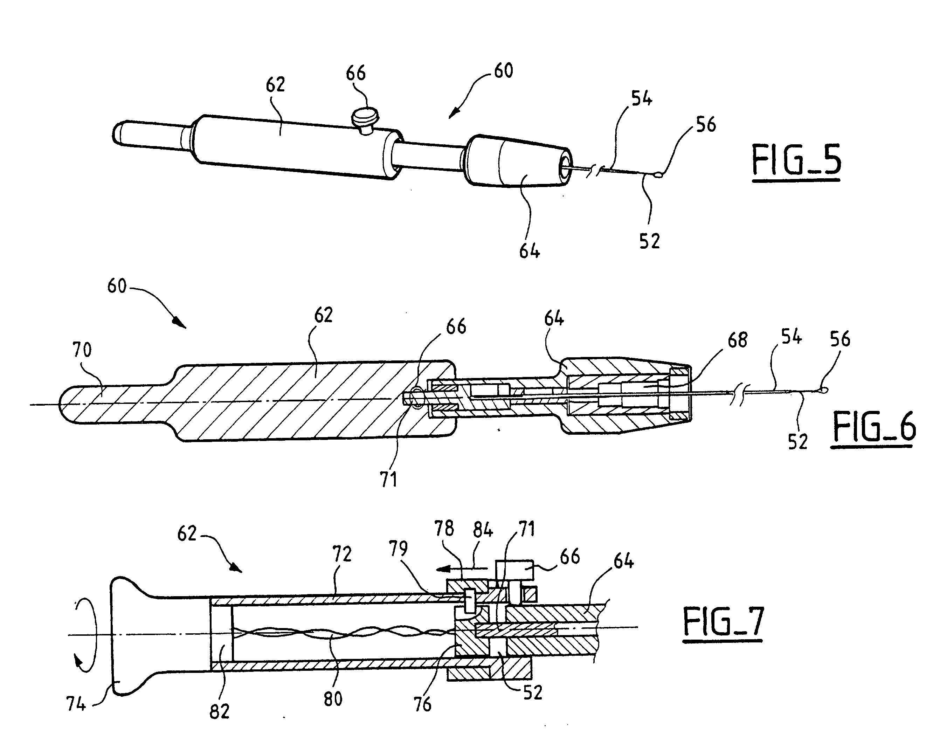

Le mouvement de rotation peut être transmis à l'âme centrale de diverses manières, soit par un outil simple, classique, semblable à un clé plate entraînant par des pans homologues une poignée solidaire de l'âme centrale, soit par un outil spécifique, par exemple celui illustré sur les figures 5, 6 et 7.

Cet outil 60 comporte deux éléments mobiles en rotation axiale l'un par rapport à l'autre, à savoir une poignée 62 destinée à entraîner en rotation l'âme centrale 52, et un manche 64 destiné à maintenir fixe le tube creux 54. Une vis de blocage 66 permet de solidariser à la poignée 62 l'extrémité de l'âme centrale émergeant en direction proximale. Le manche 64, quant à lui, comporte un logement 68 susceptible de recevoir le connecteur de liaison monté à l'extrémité proximale du corps de sonde. Ce connecteur est monté à force dans le logement 68 de manière que le manche 64 permette la rotation du corps de sonde et le maintien du tube creux 54. Par de légers mouvement du manche 64, si le mandrin a été coudé le chirurgien peut procéder à un "mapping" et à une recherche de la position optimale de la sonde sous amplificateur de brillance en orientant l'extrémité coudée vers la position recherchée. Une fois cette position trouvée, il fait tourner la poignée 62, avantageusement par l'extrémité rétrécie 70 qui peut être aisément manipulée entre le pouce et l'index, de manière à déployer la vis (dans le cas d'une vis rétractable) et à visser cette dernière dans la paroi de l'endocarde.

Le manche 64 peut être avantageusement pourvu de contacts électriques assurant une liaison avec les bornes du connecteur introduit dans le logement 68, de manière à permettre un certain nombre de mesures électriques avant l'implantation ou en cours d'implantation, par exemple des mesures d'impédance ou de seuil de capture. Un second contact électrique peut être établi pour une connexion sur l'élément 71 par l'intermédiaire de l'élément 66.

Dans une variante illustrée figure 7, la poignée 62 est munie de moyens moteurs permettant d'entraîner automatiquement et rapidement l'âme centrale 52 du mandrin.

À cet effet, la poignée 62 est constituée d'un corps fixe 72 terminé à son extrémité proximale par un embout 74 formant remontoir, et à son extrémité distale par une pièce de liaison 76 solidaire d'une bague externe 78. Un cylindre radial 79 engagé dans la pièce de liaison 76 empêche la rotation de cette pièce 76 et, par là même, de l'élément 71 et donc de l'âme 52.

Un moyen formant ressort de torsion, par exemple un ruban élastique 80, relie le remontoir 74 à la pièce de liaison 76. Le ressort est mis en tension en usine, ou par le chirurgien avant implantation ou relocalisation de la sonde. Ceci est effectué en tournant le remontoir 74, ce qui a pour effet de vriller le ruban élastique 80 et de mettre celui-ci sous contrainte de torsion.

Pour éviter tout risque d'endommagement en cas de rotation excessive du remontoir 74, il est prévu un mécanisme 82 de limitation de couple interposé entre le corps 72 et le remontoir 74. Il s'agit par exemple d'un mécanisme du même type que celui associé à une couronne de montre, dans le domaine de l'horlogerie. En cas de rotation au-delà d'un certain nombre de tours, le remontoir 74 se désolidarise du corps 72, tout en restant en tension, et le chirurgien perçoit qu'il a atteint ce point par la perception du ressaut d'un cliquet de ce mécanisme.

Une fois la tête de sonde positionnée à l'endroit voulu, le chirurgien recule la bague 78 de quelques millimètres (flèche 84) ; le cylindre radial 79 se dégage alors de la pièce de liaison 76, ce qui libère le ruban 80 et déclenche la rotation de l'élément 71 et, par voie de conséquence, de l'âme centrale 52. Ce mouvement de rotation rapide sur plusieurs tours est particulièrement avantageux car il permet un ancrage rapide et efficace de la vis, sans risque de délogement en cours de vissage.

## Revendications

1. Un nécessaire de mise en place d'une sonde intracardiaque de stimulation ou de défibrillation du type sonde à vis,
cette sonde comportant un corps de sonde (10) avec une gaine creuse souple (12) pourvue à son extrémité distale d'une tête de sonde (14) comportant au moins une électrode de stimulation et une vis (16) d'ancrage, la tête de sonde étant apte à être entraînée en rotation (28) pour permettre l'ancrage de la vis par vissage dans la paroi de l'endocarde,
ce nécessaire comportant un mandrin amovible (50), apte à être introduit à l'intérieur de la gaine du corps de sonde et mobile en translation et en rotation à l'intérieur de celle-ci, ce mandrin comportant à son extrémité distale un moyen (56) de couplage en rotation pour permettre ledit entraînement en rotation (28) de la tête de sonde,
ce nécessaire étant **caractérisé en ce que** ledit mandrin (50) comprend
- une âme centrale (52, 71) portant à son extrémité distale ledit moyen (56) de couplage en rotation, et à son extrémité proximale un organe de manipulation (62), et
- un tube creux (54) logeant axialement l'âme, la longueur de ce tube creux étant inférieure à celle de l'âme, de sorte que cette dernière émerge du tube creux aux deux extrémités, proximale et distale, du tube, de manière à permettre l'entraînement en rotation de l'âme et corrélativement du moyen de couplage en rotation, par action sur l'organe de manipulation, essentiellement sans mise en rotation du tube creux.

2. Le nécessaire de la revendication 1, dans lequel le moyen de couplage en rotation est un méplat (56) formé à l'extrémité de l'âme (52).

3. Le nécessaire de la revendication 1, dans lequel le matériau de l'âme est choisi de manière que le moment de torsion susceptible d'être transmis par l'âme soit supérieur au couple minimal d'entraînement du mécanisme de manoeuvre de la tête sur la totalité de la course de ce mécanisme, et inférieur au couple résistant opposé par la tête de sonde après ancrage de la vis dans la paroi de l'endocarde,
de manière que, en fin de course, après un nombre prédéterminé de tours correspondant à la manoeuvre complète de déploiement et de vissage de la vis d'ancrage, toute rotation supplémentaire de l'âme se traduise par un vrillage de celle-ci à l'intérieur du tube, essentiellement sans augmentation du couple transmis au moyen de couplage en rotation.

4. Le nécessaire de la revendication 1, dans lequel l'âme centrale et le tube creux sont relativement rigides par rapport à la gaine du corps de sonde, et plastiquement déformable localement.

5. Le nécessaire de la revendication 1, dans lequel l'organe de manipulation est une poignée de manipulation (62) solidaire de l'âme à son extrémité proximale.

6. Le nécessaire de la revendication 1, comportant en outre un outil de manipulation (60) comportant d'une part une poignée (62), formant ledit organe de manipulation, pourvue de moyens (66) propres à pincer localement l'âme (52, 71) de manière à solidariser cette dernière à la poignée, et d'autre part un manche (64) apte à être solidarisé à la gaine du corps de sonde et au tube creux (54).

7. Le nécessaire de la revendication 6, dans lequel la poignée inclut des moyens moteurs (74-82) aptes à permettre l'entraînement en rotation, par rapport au manche (64), desdits moyens (66) propres à pincer localement l'âme (52, 71).

8. Le nécessaire de la revendication 7, dans lequel les moyens moteurs sont des moyens à ressort élastique (80), la poignée incluant un remontoir (74) apte à mettre en tension ces moyens à ressort, ainsi que des moyens (82) de limitation du couple applicable à l'âme (52, 71) lors de la manoeuvre du remontoir.

## Claims

1. A kit for installing an intracardiac stimulation or defibrillation lead of the screw-in lead type,
said lead comprising a lead body (10) having a flexible hollow sheath (12) provided at its distal extremity with a lead head (14) comprising at least one stimulation electrode and an anchoring screw (16), the lead head being able to be driven in rotation (28) to allow the anchoring of the screw by screwing in the wall of the endocardium,
said kit including a removable stylet (50), able to be introduced inside the sheath of the lead body and mobile in translation and rotation therein, said stylet having at its distal extremity a means (56) for coupling in rotation to allow said driving in rotation of the lead head,
said kit being **characterised by** said stylet (50) comprising :
- a central core (52, 71) carrying at its distal extremity said means (56) for coupling in rotation, and at its proximal extremity a handling member (62), and
- a hollow tube (54) axially housing the core, the length of said hollow tube being shorter than the length of the core, so that the latter emerges from the hollow tube at its two extremities, proximal and distal, in order to enable the core and correlatively of the means for coupling in rotation to be driven in rotation, by action on the handling member, essentially without setting in rotation the hollow tube.

2. The kit as in claim 1, wherein the means for coupling in rotation is a flat part (56) formed at the extremity of the core (52).

3. The kit as in claim 1, wherein the material of the core is selected so that the torque likely to be transmitted by the core is higher than the minimal torque for driving the mechanism for handling the head on the totality of the displacement of this mechanism, and lower than the resistive torque opposed by the lead head after anchoring of the screw in the wall of the endocardium,
so that, at the end of the displacement, after a predetermined number of turns corresponding to the complete operation of deployment and screwing of the anchoring screw, any additional rotation of the core results in a twisting of the core inside the tube, essentially without increasing the torque transmitted by the means for driving in rotation.

4. The kit as in claim 1, wherein the central core and the hollow tube are relatively rigid as compared to the sheath of the lead body, and locally plastically deformable.

5. The kit as in claim 1, wherein the handling member is a manipulation handle (62) secured to the core at its proximal extremity.

6. The kit as in claim 1, further comprising a handling tool (60) comprising on the one hand a handle (62), forming said handling member, provided with means (66) suitable to locally grip the core (52, 71) so as to secure the latter with the handle, and on the other hand a sleeve (64) able to be connected to the sheath of the lead body and to the hollow tube (54).

7. The kit as in claim 6, wherein the handle includes motor means (74-82) adapted to enable the driving in rotation, relative to the sleeve (64), of said means (66) suitable to locally grip the core (52, 71).

8. The kit as in claim 7, wherein the motor means are means with an elastic spring (80), the handle including a winder (74) able to tension said means with a spring, as well as means (82) for limiting the torque applicable to the core (52, 71) during the operation of the winder.

## Patentansprüche

1. Gerät zum Anbringen einer Innerherzsonde zur Stimulation oder zur Defibrillation vom Typ einer schraubbaren Sonde,
wobei diese Sonde einen Sondenkörper (10) umfasst mit einer hohlen, nachgiebigen Hülse (12), versehen an seinem distalen Ende mit einem Sondenkopf (14), der wenigstens eine Stimulationselektrode und eine Verankerungsschraube (16) umfasst, wobei der Sondenkopf in der Lage ist, in Rotation (28) mitgezogen zu werden, um die Verankerung der Schraube durch Schrauben in die Endokardwand zu erlauben,
wobei das Gerät einen versetzbaren Dorn (50) umfasst, eingerichtet, ins Innere der Hülse des Sondenkörpers eingeführt zu werden, und beweglich in Translation und in Rotation im Inneren derselben, wobei dieser Dom an seinem distalen Ende ein Mittel (56) der Kupplung in Rotation umfasst, um das Mitziehen in Rotation (28) des Sondenkopfes zu erlauben,
wobei das Gerät **dadurch gekennzeichnet ist, dass** der Dorn (50) umfasst:
- einen Mittelsteg (52, 71), der an seinem distalen Ende das Mittel (65) der Kupplung in Rotation und an dessen proximalem Ende ein Manipulationsteil (62) trägt,
- ein Hohlrohr (54), das axial den Steg beherbergt, wobei die Länge dieses Hohlrohrs geringer ist als diejenige des Steges, so dass der letztere an den beiden Enden des Hohlrohrs, proximal und distal, herausragt, um das Mitziehen in Rotation des Steges und entsprechend des Mittels der Kupplung in Rotation zu erlauben, durch Einwirkung auf das Manipulationsteil, im Wesentlichen ohne das Hohlrohr in Rotation zu versetzen.

2. Gerät nach Anspruch 1, in welchem das Mittel zur Kupplung in Rotation ein Flachteil (56) ist, das am Ende des Steges (52) gebildet ist.

3. Gerät nach Anspruch 1, in welchem das Material des Steges so ausgewählt ist, dass das Torsionsmoment, das über den Steg übertragen werden kann, größer als das minimale Drehmoment zum Mitziehen des Manövriermechanismus des Kopfes über die Gesamtheit des Laufes dieses Mechanismus ist, und geringer als das Drehmoment, welches von dem Sondenkopf entgegengewirkt verbleibt, nach Verankerung der Schraube in die Endokardwand,
derart, dass, am Ende des Laufes, nach einer vorherbestimmten Anzahl an Umdrehungen, welche dem vollendeten Manöver des Entfaltens und der Verschraubung der Verankerungsschraube entspricht, sich jede zusätzliche Rotation des Steges in einer Verwindung desselben im Inneren des Rohres ausdrückt, im Wesentlichen ohne Erhöhung des Drehmoments, das an die Mittel zur Kupplung in Rotation übertragen wird.

4. Gerät nach Anspruch 1, in welchem der Mittelsteg und das Hohlrohr relativ steif sind im Verhältnis zur Hülse des Sondenkörpers, und lokal plastisch verformbar.

5. Gerät nach Anspruch 1, in welchem das Manipulationsteil ein Manipulationsgriff (62) ist, der mit dem Steg an dessen proximalen Ende fest verbunden ist.

6. Gerät nach Anspruch 1, welches außerdem ein Manipulationswerkzeug (60) umfasst, welches einerseits einen Griff (62) umfasst, welcher das Manipulationsteil bildet, versehen mit Mitteln (66), die dazu geeignet sind, den Steg (52, 71) lokal zu klemmen, um den letzteren mit dem Griff fest zu verbinden, und andererseits einen Schaft (64), der eingerichtet ist, mit der Hülse des Sondenkörpers und mit dem Hohlrohr (54) fest verbunden werden zu können.

7. Gerät nach Anspruch 6, in welchem der Griff Motormittel (74-82) einschließt, die geeignet sind, das Mitziehen in Rotation, im Verhältnis zum Schaft (64), der Mittel (66), die geeignet sind, den Steg (52, 71) lokal zu klemmen, zu erlauben.

8. Gerät nach Anspruch 7, in welchem die Motormittel elastische Federmittel (80) sind, wobei der Griff eine Aufziehvorrichtung (74) enthält, die geeignet ist, diese Federmittel in Spannung zu versetzen, sowie Mittel (82) zur Begrenzung des auf den Steg (52, 71) anwendbaren Drehmoments bei der Betätigung der Aufziehvorrichtung.
